# EUROPEAN PATENT APPLICATION

(11) **EP 1 738 770 A1**
(43) Date of publication of application: **03.01.2007**
(21) Application number: 06397013.1
(22) Date of filing: 13.06.2006
(51) Int. Cl.: A61K 47/48

(54) **Method for synthesis of phospholipids-PEG-biomolecule conjugates**

(30) Priority: 30.06.2005 FI 20050695
(71) Applicant: CTT Cancer Targeting Technologies OY, 00790 Helsinki (FI)
(72) Inventor: Simpura, Ilkka, FI-00920, HELSINKI (FI)
(74) Representative: Matilainen, Mirja Helena

(57) **Abstract**

The present invention relates to methodology of manufacturing phospholipids-PEG-biomolecule conjugates suitable for use as a component of diagnostic or therapeutic liposomes/micelles in targeted diagnostics or therapy and concerns specifically a simple method of synthesis, purification and analysis of phospholipid biomolecule, e.g. peptide, conjugates. The invention thus provides micellar peptides, which can be used for improving targeting of liposomes/micelles to tumour cells, for enhancing the uptake of liposomes by tumour cells, and for selected liposomal delivery of chemotherapeutic agents into tumour cells.

## Description

### Field of the Invention

The present invention relates to methodology of manufacturing phospholipids-PEG-biomolecule conjugates suitable for use as a component of diagnostic or therapeutic liposomes/micelles in targeted diagnostics or therapy and concerns specifically a simple method of synthesis, purification and analysis of phospholipid biomolecule, e.g. peptide, conjugates. The invention thus provides micellar peptides, which can be used for improving targeting of liposomes/micelles to tumour cells, for enhancing the uptake of liposomes by tumour cells, and for selected liposomal delivery of chemotherapeutic agents into tumour cells.

### Background of the Invention

In chemotherapy, only a fraction of the drug reaches cancer cells, whereas the rest of the drug may damage normal tissues. Adverse effects can be reduced by the administration of cancer drugs encapsulated in liposomes¹. Improved liposome compositions have been described, so as to enhance their stability and to prolong their lifetime in the circulation². Among such compositions, phospholipids conjugated to monomethoxy polyethylene glycol (PEG) have been widely used since 1984 when Sears coupled, via an amide link, carboxy PEG and purified soya phosphatidyl ethanolamine (PE)³. The addition of PEG onto the liposome surface attracts a water shell surrounding the liposome. This shell prevents the adsorption of various plasma proteins (opsonins) to the liposome surface so that liposomes are not recognized and taken up by the reticulo-endothelial system. Enhanced selectivity can be obtained by attaching to the surface of the liposome specific antibodies or small peptides recognizing plasma membrane antigens of the target cell, thus augmenting the uptake of the liposome by the cell⁴. The current awareness of targeting liposomes has grown significantly during the recent decade. The recent advances of targeted liposomes have been recently reviewed⁵.

There exist several synthetic techniques to provide phospholipids-PEG-biomolecule conjugates. Said conjugates can be made in two different ways. Firstly a suitably functionalized phospholipids-PEG molecule is incorporated to a pre-made liposome and then it is incubated with a biomolecule (antibody, peptide, etc.). In this approach the biomolecule should be suitably functionalized which is one of the drawbacks of this method. Secondly the whole construct phospholipids-PEG-biomolecule is synthesized before it is incorporated to a liposome. The benefits of this method include that the conjugate can be analyzed before incorporation which can not be done in the previous method. On the other hand the conjugate also needs to be chromatographically purified.

The synthetic methods presented for the preparation of phospholipids-PEG-biomolecule conjugate can also be divided into two categories based on the reaction medium used. They are liquid and solid phase methods. Liquid phase methods can be performed in an organic solvent (halogenated or dimethylformamide) or buffered water. Reaction partners (phospholipids-PEG and biomolecule, e.g. peptide) should have proper functionalities in order to avoid undesirable side reactions. Common reaction partners are activated ester vs. primary amino functionality (formation of amide bond)⁶ and electron withdrawing group conjugated double bond vs. thiol functionality (formation of thioether via Michael addition)⁷. There exist few examples of solid phase peptide synthesis of phospholipids-PEG-peptides⁸. They are in general more complicated and time consuming reactions than corresponding reactions in liquid phase due to the slower reactivity of PEG in solid phase.

Phospholipid-PEG-peptide conjugates need to be purified after the coupling reaction. Several chromatographic methods have been used for purification. Phospholipid-PEG-biomolecule conjugates can be purified by silica gel, reverse phase silica or by size exclusion chromatography and dialysis is also used depending on the nature of the biomolecule/peptide. Silica gel chromatography is based on hydrophilic interaction between stationary phase and the elute and the reverse phase is based on hydrophobic interactions. Size exclusion chromatography is based on the resolution of molecules by the size so that the biggest molecules come out from the column first. Phospholipid-PEG-peptide conjugates are amphiphilic and tend to form micelles in aqueous solution and they are firstly eluted from the column.

### Summary of the Invention

We describe here an improved method for the synthesis of phospholipids-PEG-biomolecule conjugates. Reaction variables used in the method according to the invention like the ratio of starting materials and reaction rate accelerators have been defined at the screening stage. Optimal reaction conditions can be transferred to larger scale reactions. Further, a simple and effective isolation method of the product has been developed. Derivatization of the product enables chromatographic analysis for both monitoring the progress of reaction and analysis of the product.

### Detailed Description of the Invention

The present invention describes an improved process for manufacturing ofphospholipids-PEG-peptide conjugates. Said manufacturing process for preparing a phospholipids-PEG-peptide conjugate may comprise the following steps
1. Small scale optimization step of the coupling reaction between pegylated phospholipids and peptide.
2. Chromatographic analysis of data from the optimization step by derivatization of phospholipids-PEG-peptide conjugate.
3. Insertion of the optimized reaction parameters to a larger scale synthesis.
4. Purification of phospholipids-PEG-peptide conjugate by precipitation procedure and by HPLC if needed.
5. Chromatographic analysis of the product purity by derivatization of phospholipids-PEG-peptide conjugate.

Information from the optimization step, analysis step and purification step can be transferred to larger process scale.

At the optimization step small scale synthesis was performed using several variables of reaction, for example excess of acylating reagent and reaction additives. Results from these optimization reactions were obtained by C18-RP-HPLC analysis after derivatization of phospholipids-PEG-peptide conjugates. After reaction conditions were obtained from the analysis of optimization step the reaction could be performed in a larger scale. The outcome of the reaction can be analyzed using developed HPLC method and purified by a efficient precipitation step which separates non-reacted phospholipids-PEG from phospholipids-PEG-peptide conjugate.

The method according to the present invention for preparing a phospholipids-PEG-biomolecule conjugate comprises the steps of coupling pegylated phospholipids and a biomolecule by covalent attachment and purifying the obtained conjugate, and is characterized in that pegylated phospholipids are used in excess compared to the amount of the biomolecule, the coupling step is accelerated by the addition of inorganic additives, and the obtained phospholipids-PEG-biomolecule conjugate is purified by precipitation procedure.

In a most preferable embodiment of the invention the biomolecule is any peptide having one free primary amino functionality to be connected covalently to carboxy functionality of phospholipids-PEG-COOH. The peptide is first covalently attached (coupled) to the end group of the poly(ethylene glycol) polymer chain of the PEG phospholipids, DSPE-PEG-NHS. Preferred peptides used in the method according to the invention are (E-cyclo-(RGDfK)₂), GRENYHGCTTHWGFTLC-NH₂, K(DOTA)RENYHGCTTHWGFTLC-NH₂, Ac-GRENYHGCTTHWGFTLCK-NH₂, YQGDAHGDDDEL and YADGAC₁₋₈PC₃₋₉FLLGCC peptides

We have found out that the addition of non-soluble inorganic additives in excess to the reaction mixture accelerates coupling reactions remarkably. For example, if the reaction does not progress due to a steric hindrance, inorganic additives can be used to accelerate the formation of the product. There exists one example in literature of the use of equimolar amount of these salts in a similar reaction but the effect of positive acceleration was not mentioned.⁹ The exact role of these non-soluble additives as an accelerator of the reaction rate is not exactly known. If they are compared to organic additives like DMAP (dimethylaminopyridine), which is a nucleophilic base and is commonly used in synthesis of esters and amides, inorganic additives do not work in a similar way. One possible reason to reaction activation is the formation of weak Lewis acid-base adducts which open the tertiary structure of the peptides and enable the reaction between amine and acyl functionalities.

The inorganic additives used in the method according to the present invention are most preferably a mixture of an inorganic base and an inorganic drying agent. Suitable inorganic bases include for example carbonates or bicarbonates of alkali metals, alkaline earth metals or lanthanides, among which alkali and alkaline earth metal carbonates lithium carbonate, sodium carbonate and potassium carbonate are preferred. Suitable inorganic drying agents are sulfates of alkali metals and alkaline earth metals, preferably sodium sulfate and magnesium sulfate.

The ratio of starting materials may vary from equimolar to tens of molar equivalents of phospholipids-PEG compared to the amount of the biomolecule. The amount of inorganic additives may be from tens to hundred of molar equivalents of the biomolecule. Using the excess of DSPE-PEG-NHS and inorganic additives when needed the reaction can be driven to the end by consuming the starting material peptide.

Excess DSPE-PEG-NHS is removed from the product by a simple repeated precipitation procedure. Initial precipitation is carried out by adding to the reaction mixture a suitable solvent or solvent mixture as defined below. The raw material of reaction is then dissolved in a suitable alcohol, such as methanol, ethanol, n-propanol, i-propanol, n-butanol, 2-butanol or t-butanol. The separation of non reactive phospholipids-PEG from phospholipids-PEG-peptide conjugate is then performed by an appropriate solvent or solvent mixture, such as a suitable alkylether or any other solvent which forms one phase with the alcohol used and is suitably hydrophobic in order to precipitate the phospholipids-PEG-peptide when the molar excess of phospholipids-PEG stays at solvent phase. An appropriate solvent or solvent mixture precipitates phospholipids-PEG-peptide conjugate from the alcohol solution and the product can be isolated. This precipitation procedure avoids the usage of costly and time consuming chromatographic methods for product purification. After the purification by precipitation the product may be dissolved in a suitably buffered water solution, freezed and lyophilized.

Earlier the reaction mixture was precipitated from dimethylformamide by diethyl ether and the residual solid material was redissolved in dimethylformamide and the diethyl ether precipitation was repeated¹⁰. One advantage of the new modified precipitation procedure according to the present invention is that the residual dimethylformamide can be removed from the solid product by alkyl alcohol in redissolving steps. Also smaller volumes of diethyl ether are needed for product precipitation because alcohols are poorer solubilizers ofpeptides of interest than dimethylformamide. Further, the fact that dimethylformamide is non volatile and can not be removed by lyophilization makes the use of volatile alkyl alcohols an advantage of this method.

The coupling reaction between peptide and DSPE-PEG-NHS can be monitored and the purity of the products identified by C 18-RP-HPLC after the basic saponification of a small sample from the precipitated reaction mixture. We found out that basic hydrolysis of diacyl esters reduces the hydrophobicity of the compound so that hydrolyzed residual PEGylated-peptide can be analyzed using normal C-18 reverse phase chromatography. This combination ofpurification and analysis steps is cost-effective and precise methodology for synthesis ofphospholipid-PEG-peptide conjugates. It can be applied to any phospholipids and peptides.

### Brief Description of the Drawings

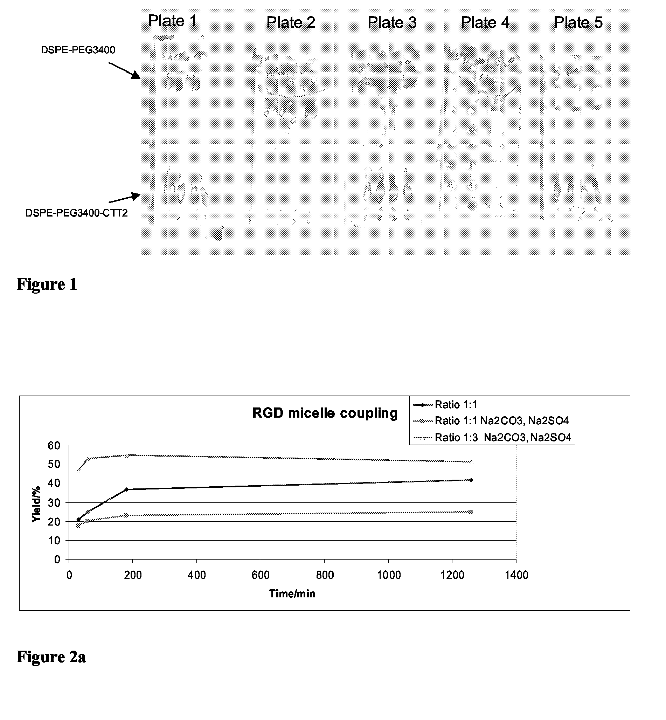

**Figure 1.** Thin layer chromatography (TLC) analysis of the purification process by precipitation of DSPE-PEG3400-CTT2 (example 1). **Plate 1,** TLC of the raw reaction mixture; **Plate 2,** Supernatant of the first precipitation (MeOH: Et₂O, 1:4); **Plate 3,** The pellet suspension of the first precipitation dissolved in MeOH; **Plate 4,** Supernatant of the second precipitation (MeOH: Et₂O 1:4); **Plate 5,** The pellet suspension of the second precipitation dissolved in MeOH.

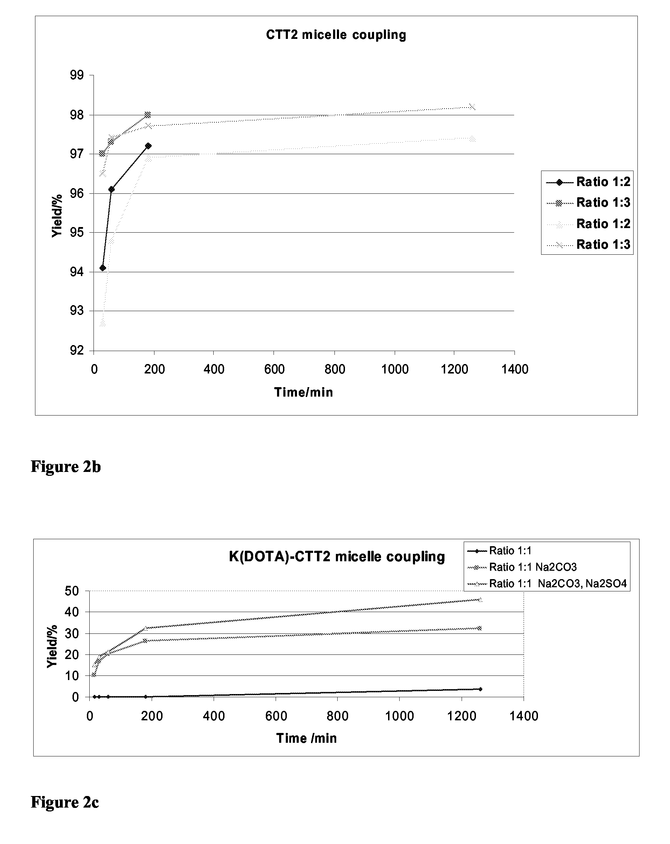

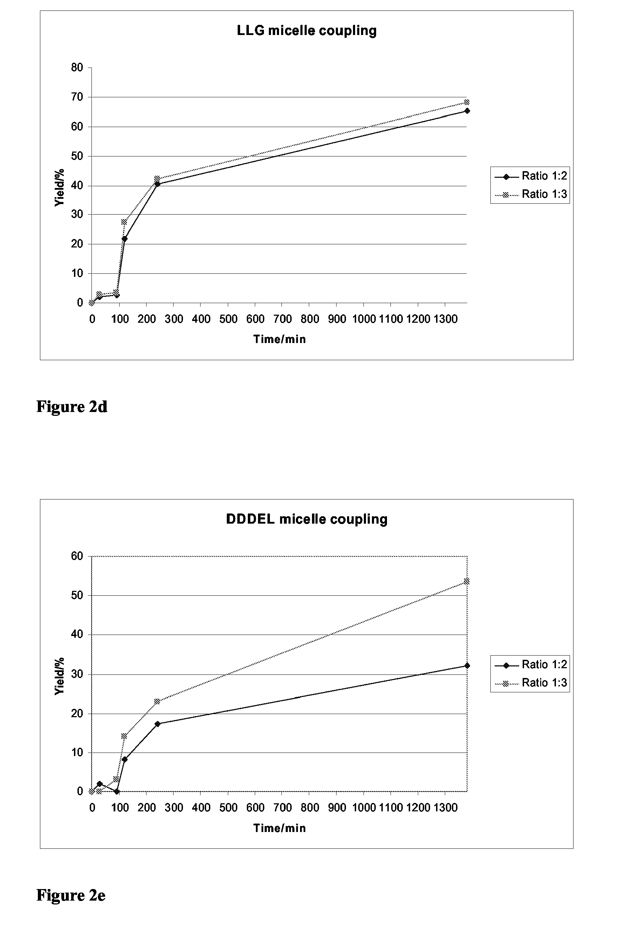

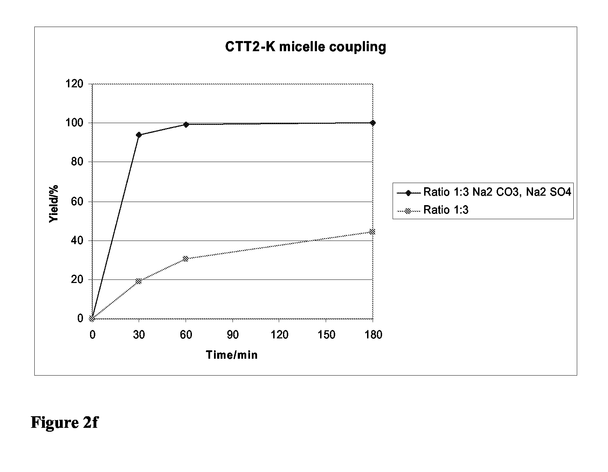

**Figure 2.** The RP-HPLC analysis of example reactions. The ratio of DSPE-PEG3400 coupled peptide versus non coupled peptide is presented on y-axis as a function of time (x-axis).
   a) Example 1; The effect of inorganic additives and the ratio of starting material were examined for RGD (E-cyclo-(RGDfK)₂) peptide coupling to DSPE-PEG3400-NHS.
   b) Example 3; The ratio of starting material and reaction time were examined for CTT2 (cyclo-GRENYHGCTTHWGFTLC-NH₂) peptide coupling to DSPE-PEG3400-NHS.
   c) Example 4; In this procedure, the effect of inorganic additives were examined for K(DOTA)-CTT2 (cyclo-K(DOTA)RENYHGCTTHWGFTLC-NH₂) peptide coupling to DSPE-PEG3400-NHS.
   d) Example 6; The effect of inorganic additives and the ratio of starting material were examined for LLG (bicyclo-YADGAC₁₋₈PC₃₋₉FLLGCC) peptide coupling to DSPE-PEG3400-NHS.
   e) Example 8; The effect of inorganic additives and the ratio of starting material were examined for DDDEL (YQGDAHFDDDEL) peptide coupling to DSPE-PEG3400-NHS.
   f) Example 10; the effect of inorganic additives was examined for CTT2K (cyclo-Ac-GRENYHGCTTHWGFTLCK-NH₂) peptide coupling to DSPE-PEG3400-NHS.
**Figure 3.** Molecular structure of DSPE-PEG3400-CTT2 peptide.
**Figure 4**. Peptides used in this study for coupling reaction to pegylated phospholipids.
**Figure 5.** Thin layer chromatography (TLC) analysis of the basic hydrolysis of DSPE-PEG3400-CTT2 conjugate. **Lane 1,** TLC of the DSPE-PEG3400-CTT2 conjugate; **Lane** 2, Basic hydrolysis of DSPE-PEG3400-CTT2 conjugate; **Lane 3,** Empty lane; **Lane 4**, Combination of 1 and 2 lanes.

### Abbreviations:

- CTT2: amidated cyclic GRENYHGCTTHWGFTLC peptide
- CTT2K: amidated cyclic Ac-GRENYHGCTTHWGFTLCK peptide
- K(DOTA)CTT2: amidated cyclic K(DOTA)RENYHGCTTHWGFTLC-NH₂peptide
- DDDEL: YQGDAHGDDDEL peptide
- LLG: bicyclo-YADGAC₁₋₈PC₃₋₉FLLGCC peptide
- DMAP: dimethylaminopyridine
- DOTA: 1,4,7,10-tetraazacyclododecane-1,4,7,10-tetraacetate
- DSPE-PEG-NHS: 1,2-Distearoyl-*sn*-Glycero-3-Phosphoethanolamine-*n-*[poly(ethylene glycol)]-*N*-hydroxysuccinamidyl carbonate
- HPLC: high-performance liquid chromatography
- PE: phosphatidyl ethanolamine
- PEG: polyethylene glycol
- RP-HPLC: reverse phase high-performance liquid chromatography
- TLC: thin-layer chromatography
- TOF-MALDI MS: time of flight matrix-assisted laser desorption/ionization mass spectrometer

### Experimental

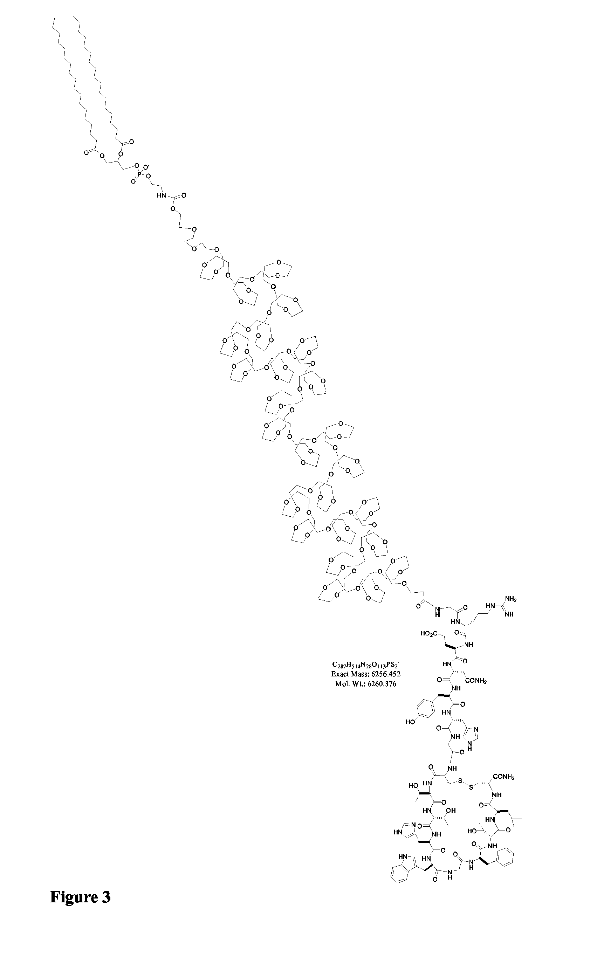

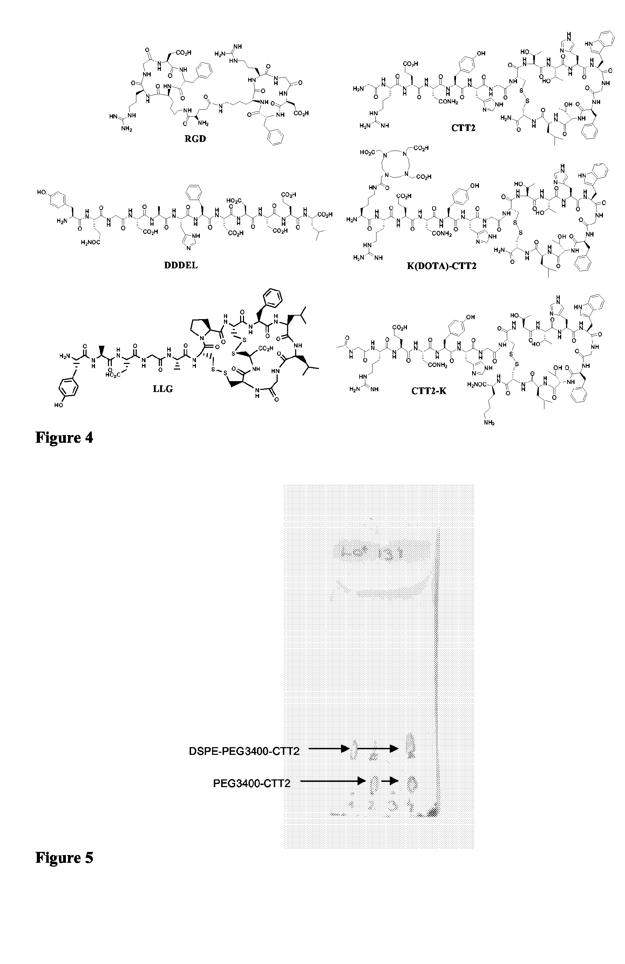

Peptides used in the examples are all originally found by phage display technique. They are chosen to cover the structural diversity of peptides. The peptides K(DOTA)-CTT2 and CTT2-K are derivatives of CTT2 peptides. They are presented in Table 1 and the molecular structures are presented in Figure 4. Molecular structure of DSPE-PEG3400-CTT2 is presented in Figure 3.

**Table 1: Peptides used in this study and their targets**

| Peptide | Target |
|---|---|
| RGD | α_{V}β₃ integrin |
| CTT2¹⁰ | MMP 9 |
| LLG¹¹ | α_{M}β₂ integrin |
| DDDEL¹² | α_{M}β₂ integrin |

### Example 1. Optimization of peptide coupling reaction: DSPE-PEG3400-RGD

In this procedure, the effect of inorganic additives and the ratio of starting material were examined for RGD (E-cyclo-(RGDfK)₂) peptide coupling to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Equimolar ratio of the peptide and the PEG phospholipid, DSPE-PEG3400-NHS was used in reaction 1 and 2. In reaction 3 three equivalents of DSPE-PEG3400-NHS were used. Sodium carbonate and sodium sulfate were added to reaction 2 and 3.

RGD peptide 1.1 mg (1 eq.) and DSPE-PEG3400-NHS 6.1 mg (1.67 eq.) were separately dissolved in 600 µl of dimethylformamide. RGD peptide solution was divided to three vials 200 µl each. To vials 1 and 2 were added 150 µl of DSPE-PEG3400-NHS solution and 300 µl to vial 3. Inorganic additives were added as follows: To vial 2 were added 7.5 mg of sodium carbonate and 3.1 mg of sodium sulfate. To vial 3 were added 8 mg of sodium carbonate and 5.3 mg of sodium sulfate. Reaction was stirred at room temperature.

Samples 25 µl by volume were taken from all the reactions at timepoints 30, 60 and 180 minutes and 21 hour after the beginning of the reaction. Reactions were quenched after 21 hour. Samples were precipitated by addition of diethylether and centrifuged 13200 rpm 10 min. Supernatant was poured away and the solid residue was set on -70°C.

Samples were dissolved in 100 µl of methanol and 25 µl of 1 M sodium hydroxide were added. After two hours 250 µl of 1% TFA in water was added to samples and after centrifugation samples were analyzed by C-18 RP-HPLC (Figure 2a).

### Example 2. Larger scale peptide coupling reaction:

### DSPE-PEG3400-RGD

In this procedure, RGD (E-cyclo-(RGDfK)₂) peptide was covalently attached to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Different molar ratios of the peptide and the PEG phospholipid, as well as the reaction time were tested to optimize the coupling reaction.

RGD peptide 10 mg (1 eq.) DSPE-PEG3400- NHS 97.1 mg (3 eq.) was dissolved in 2.5 ml of dimethylformamide. Reaction mixture was shaken overnight at room temperature.

### Purification

Reaction mixtures were precipitated by addition of diethylether ten times the volume of reaction. After centrifugation 4200 rpm 20 min the solid residues were dissolved in methanol 2 ml. Diethylether 8 ml were added to product containing methanol fraction and product precipitates. After centrifugation (1000 rpm 20 min) the supernatant was poured away and the precipitation step was repeated. After second precipitation solid residue was dissolved in water, freezed and lyophilized. Product 33.4 mg was obtained as a white solid.

### Monitoring the purity of the product

Samples (25 µl by volume) were taken from all the reaction mixtures just before the quenching of the reaction. Samples were precipitated by addition of diethylether and centrifuged 13200 rpm 10 min. Supernatant was poured away, the solid residue was dissolved in 100 µl of methanol and 25 µl of 1 M sodium hydroxide were added. After two hours 250 µl of 1% TFA in water was added to samples and after centrifugation samples were analyzed by C 18 RP-HPLC. Purity of the hydrolyzed product was 98 % determined by C18-RP-HPLC.

### Example 3. Optimization of peptide coupling reaction:

### DSPE-PEG3400-CTT2

In this procedure, CTT2 (cyclo-GRENYHGCTTHWGFTLC-NH₂) peptide was covalently attached to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Different molar ratios of the peptide and the PEG phospholipid, as well as the reaction time were tested to optimize the coupling reaction.

8.8 mg of CTT2 peptide was dissolved in 2 ml of dimethylformamide. DSPE-PEG3400-NHS was dissolved in 2 ml of dimethylformamide. CTT2 peptide solution was divided to four reaction vessels 500 µl each followed by addition of DSPE-PEG3400-NHS solution so that 400 µl of solution was added to vessels 1 and 2 and 600 µl to vessels 3 and 4. Additional 200 µl of DMF was added to reaction vessels 1 and 2 in order to equalize the concentration of peptide in reaction vessel.

Samples of 25 µl by volume were taken from all the reactions at timepoints 30, 60 and 180 minutes after the beginning of the reaction. Reactions 1 and 2 were quenched after three hours and reactions 3 and 4 after 21 hour. Additional samples from reactions 3 and 4 were also taken at 21 h. Samples were precipitated by addition of diethylether and centrifuged 13200 rpm 10 min. Supernatant was poured away and the solid residue was set on -70°C.

Samples were dissolved in 100 µl of methanol and 25 µl of 1 M sodium hydroxide were added. After two hours 250µl of 1% TFA in water was added to samples and after centrifugation samples were analyzed by C-18 RP-HPLC (Figure 2b). Reaction mixtures were precipitated by addition of diethylether ten times the volume of reaction. After centrifugation (4200 rpm 20 min), the solid residues were dissolved in 1 500µl methanol and 2 µl sample for TLC analysis was taken. 2 ml of diethylether was added to the product containing methanol fraction and product precipitates. After centrifugation 10 µl sample for TLC was taken from the supernatant. The solid residue was dissolved in methanol and precipitation repeated.

The solid residue was dissolved in 500 µl of methanol followed by addition of 2.5 ml of distilled water. Solutions were freezed at -70°C and lyophilized overnight. Products were obtained as white solids. Yields of the reactions 1, 2, 3 and 4 were 5.1, 5.6, 5.6 and 5.9 mg.

### Example 4. Optimization of peptide coupling reaction DSPE-PEG3400-K(DOTA)-CTT2

In this procedure, the effect of inorganic additives was examined for K(DOTA)-CTT2 (cyclo-K(DOTA)RENYHGCTTHWGFTLC-NH₂) peptide coupling to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Equimolar ratio of the peptide and the PEG phospholipid, DSPE-PEG3400- NHS was used in all reactions. Sodium carbonate was used as a additive in reaction 2 and sodium carbonate and sodium sulfate were added to reaction 3.

Cyclo-K(DOTA)RENYHGCTTHWGFTLC-NH₂ peptide 2.5 mg (1 eq.) and DSPE-PEG3400- NHS 4.8 mg (1 eq.) were separately dissolved in 600 µl of dimethylformamide. Peptide solution and DSPE-PEG3400-NHS solution were divided into three vials 200 µl each per compound. Inorganic additives were added as follows: To vial 2 were added 3.3 mg of sodium carbonate. To vial 3 were added 3.9 mg of sodium carbonate and 9.4 mg of sodium sulfate. Reaction was stirred at room temperature. Samples of 25 µl by volume were taken from all the reactions at timepoints 15, 30, 60 and 180 minutes and 21 hours after the beginning of the reaction. Reactions were quenched after 21 hours. The samples were precipitated by addition of diethyl ether and centrifuged 13200 rpm 10 min. The supernatant was poured away and the solid residue was set on -70°C.

Samples were dissolved in 100 µl of methanol and 25 µl of 1 M sodium hydroxide were added. After two hours 250 µl of 1% TFA in water was added to samples and after centrifugation samples were analyzed by C-18 RP-HPLC (Figure 2c).

### Example 5. Larger scale peptide coupling reaction DSPE-PEG3400- K(DOTA)-CTT2

In this procedure, K(DOTA)-CTT2 (cyclo-K(DOTA)RENYHGCTTHWGFTLC-NH₂) peptide was covalently attached to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage.

K(DOTA)-CTT2 peptide 5.6 mg (1 eq.), DSPE-peg3400- NHS 40.1 mg (4 eq.), sodium carbonate 12.9 mg and sodium sulfate 10 mg were dissolved in 1.0 ml of dimethylformamide. Reaction mixture was shaken overnight at room temperature.

### Purification

Reaction mixtures were precipitated by the addition of diethyl ether ten times the volume of reaction. After centrifugation 3000 rpm 15 min the solid residues were dissolved in 0.5 ml methanol. Diethyl ether 2.5 ml were added to product containing methanol fraction and product precipitates. After centrifugation (1000 rpm, 15 min) the solvent phase was poured away and the precipitation step was repeated. After second precipitation the solid residue was dissolved in water, freezed and lyophilized. Product 11.8 mg was obtained as a white solid. Purity of the hydrolyzed product was 92.3 % determined by C18-RP-HPLC.

### Example 6. Optimization of peptide coupling reaction: DSPE-PEG3400-LLG

In this procedure, the effect of inorganic additives and the ratio of starting material were examined for LLG (bicyclo-YADGAC₁₋₈PC₃₋₉FLLGCC) peptide coupling to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Molar ratios 1:2 and 1:3 of the peptide and the DSPE-PEG3400-NHS were used. Sodium carbonate and sodium sulfate were added to reaction mixture after 90 min starting.

LLG peptide 2.2 mg (1 eq.) and DSPE-PEG3400- NHS 17 mg (2.5 eq.) were separately dissolved in 500 µl of dimethylformamide. LLG peptide solution was divided to two vials 250 µl each. To vials 1 and 2 were added 200 and 300 µl of DSPE-PEG3400-NHS solution. Inorganic additives were added after 90 minutes from the start. To vial 1 were added 4.6 mg of sodium carbonate and 3 mg of sodium sulfate. To vial 2 were added 6,5 mg of sodium carbonate and 6,2 mg of sodium sulfate. Reactions were stirred at room temperature.

Samples 25 µl by volume were taken from all the reactions at timepoints 30, 90, 120, 240 minutes and 23 and 47 hours after the beginning of the reaction. Reactions were quenched after 23 hour. Samples were precipitated by addition of diethylether and centrifuged 13200 rpm 10 min. Supernatant was poured away and the solid residue was set on -70°C.

Samples were dissolved in 100 µl of methanol and 25 µl of 1 M sodium hydroxide were added. After two hours 250 µl of 1% TFA in water was added to samples and after centrifugation samples were analyzed by C-18 RP-HPLC (Figure 2d)

### Example 7. Larger scale peptide coupling reaction: DSPE-PEG3400-LLG

In this procedure, LLG (bicyclo-YADGAC₁₋₈PC₃₋₉FLLGCC) peptide was covalently attached to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage.

LLG peptide 5.4 mg (1 eq.), DSPE-PEG3400- NHS 50.8 mg (3 eq.), sodium carbonate 18.4 mg and sodium sulfate 8.4 mg were dissolved in 1.5 ml of dimethylformamide. Reaction mixture was shaken overnight at room temperature.

### Purification

Reaction mixtures were precipitated by addition of diethylether ten times the volume of reaction. After centrifugation 3000 rpm 15 min the solid residues were dissolved in methanol 0.5 ml. Diethylether 3 ml were added to product containing methanol fraction and product layer separates as a yellow oil. After centrifugation (1000 rpm 20 min) diethylether layer was poured away and the residual yellow oil precipitated by diethylether. Diethylether was poured away and the precipitation step was repeated. After second precipitation the solid residue was dissolved in water, freezed and lyophilized. Product 19.7 mg was obtained as a white solid. Purity of the hydrolyzed product was 92.5 % determined by C18-RP-HPLC.

### Example 8. Optimization of peptide coupling reaction DSPE-PEG3400-DDDEL

In this procedure, the effect of inorganic additives and the ratio of starting material were examined for DDDEL (YQGDAHFDDDEL) peptide coupling to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Molar ratios 1:2 and 1:3 of the peptide and the DSPE-PEG3400- NHS were used. Sodium carbonate and sodium sulfate were added to the reaction mixture starting after 90 min.

DDDEL peptide 2.2 mg (1 eq.) and DSPE-PEG3400- NHS 16,7 mg (2.5 eq.) were separately dissolved in 500 µl of dimethylformamide. DDDEL peptide solution was divided to two vials 250 µl each. To vials 1 and 2 were added 200 and 300 µl of DSPE-PEG3400-SPA solution. Inorganic additives were added after 90 minutes from the start. To vial 1 were added 6,5 mg of sodium carbonate and 6,2 mg of sodium sulfate. To vial 2 were added 8 mg of sodium carbonate and 3,2 mg of sodium sulfate. Reaction was stirred at room temperature.

Samples 25 µl by volume were taken from all the reactions at timepoints 30, 90, 120, 240 minutes and 23, 47 hour after the beginning of the reaction. Reactions were quenched after 23 hour. Samples were precipitated by addition of diethyl ether and centrifuged 13200 rpm 10 min. Supernatant was poured away and the solid residue was set on -70°C. Samples were dissolved in 100 µl of methanol and 25 µl of 1 M sodium hydroxide were added. After two hours 250 µl of 1% TFA in water was added to samples and after centrifugation samples were analyzed by C-18 RP-HPLC (Figure 2e).

### Example 9. Larger scale peptide coupling reaction DSPE-PEG3400-DDDEL

In this procedure, DDDEL (YQGDAHFDDDEL) peptide was covalently attached to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage.

DDDEL peptide 5.3 mg (1 eq.), DSPE-PEG3400- NHS 59 mg (3 eq.), sodium carbonate 22.9 mg and sodium sulfate 14.1 mg were dissolved in 1.5 ml of dimethylformamide. Reaction mixture was shaken overnight at room temperature.

### Purification

Reaction mixtures were precipitated by addition of diethylether ten times the volume of reaction. After centrifugation 3000 rpm 15 min the solid residues were dissolved in methanol 0.5 ml. Diethylether 2 ml were added to product containing methanol fraction and product precipitates. After centrifugation (1000 rpm, 15 min) the solvent phase was poured away and the precipitation step was repeated. After second precipitation solid residue was dissolved in water, freezed and lyophilized. Product 13.2 mg was obtained as a white solid. Purity of the hydrolyzed raw product was 57.6 % determined by C18-RP-HPLC. Final purification of the product was performed by SE-HPLC. Purity of the hydrolyzed SE-HPLC purified product was 95.5 % determined by C18-RP-HPLC and the yield of the product was 4.7 mg.

### Example 10. Optimization of peptide coupling reaction CTT2K-PEG3400-DSPE

In this procedure, the effect of inorganic additives was examined for CTT2K (cyclo-Ac-GRENYHGCTTHWGFTLCK-NH₂) peptide coupling to PEG phospholipids through the chemical reaction between the terminal amine of the peptide and the functional NHS (hydroxysuccinimidyl) group at the end of the poly(ethylene glycol) polymer chain of the PEG phospholipid. The reaction between the terminal amine and the active succinimidyl ester of the PEG carboxylic acid produced a stable amide linkage. Molar ratio 1:3 of the peptide and the DSPE-PEG3400- NHS was used. Sodium carbonate and sodium sulfate were added to the reaction mixture.

CTT2K peptide 1.8 mg (1 eq.) and DSPE-PEG3400- NHS 11 mg (3 eq.) were separately dissolved in 300 µl of dimethylformamide. CTT2K peptide solution was divided to two vials 250 µl each. To vials 1 and 2 were 300 µl of DSPE-PEG3400- NHS solution. To vial 1 were added 4.7 of sodium carbonate and 5.8 of sodium sulfate. Reaction was stirred at room temperature.

Samples 25 µl by volume were taken from all the reactions at timepoints 30, 60, 180 minutes and 23 hours after the beginning of the reaction. Reactions were quenched after 22 hours. Samples were precipitated by addition of diethyl ether and centrifuged 13200 rpm 10 min. Supernatant was poured away and the solid residue was set on -70°C. Samples were dissolved in 100 µl of methanol and 25 µl of 1 M sodium hydroxide were added. After two hours 250µl of 1% TFA in water was added to samples and after centrifugation samples were analyzed by C-18 RP-HPLC (Figure 2f).

### Purification

Reaction mixture from vial 1 was precipitated by the addition of diethyl ether ten times the volume of reaction. After centrifugation (3000 rpm 15 min) the solid residues were dissolved in 0.4 ml methanol. Diethyl ether 1.6 ml were added to product containing methanol fraction and product precipitates. After centrifugation (1000 rpm 15 min) supernatant was poured away and the precipitation step was repeated. After second precipitation solid residue was dissolved in water, freezed and lyophilized. Product 1.4 mg was obtained as a white solid.

### Analysis of molecular identity of phospholipids-PEG3400-peptides

The progress of coupling reaction was followed by TLC-plate and C18-RP HPLC. Purity of the products (ratio of coupled peptide to the amount of peptide originally inserted to reaction) was determined by C18-RP HPLC. Molecular masses of phospholipid-PEG3400-peptide conjugates were analyzed by TOF-MALDI MS spectrophotometer using Matrix-assisted laser desorption/ionization time-of-flight (MALDI-TOF) mass spectrometric analyses were performed using an Ultraflex TOF/TOF instrument (Bruker Daltonik GmbH, Bremen, Germany) equipped with a nitrogen laser operating at 337 nm. The mass spectra were acquired in positive ion linear and/or reflector mode using α-Cyano-4-hydroxycinnamic acid as the matrix and extemal calibration with Peptide calibration standard (Bruker part # 206195) or Protein calibration standard I (Bruker part # 206355).

Molecular weight of the whole molecule was not obtained from the MALDI-MS. Molecular fragmentation of phospholipids-PEG3400-peptide conjugates was observed in all samples. Fragmentation corresponds the cleavage of C-O bond between glycerol and phosphor diester. Formed species 1,2-propyl-di-stearyl cation was observed in every spectra. The uniformity of the product was proved by hydrolysis of DSPE-PEG3400-CTT2 peptide and observation that hydrolysis product moves more slowly at the TLC-plate (figure 5). This proves that the observed fragmentation is happened in MALDI MS. Corresponding fragmentation (cleavage ofphosphodiester) is also observed in MALDI MS analysis of DNA.¹³

**Table 2.**

| Phospholipid-PEG3400-peptide | Fragment 1 /g/mol | Fragment 2/g/mol |
|---|---|---|
| DSPE-PEG3400-(E-cyclo-(RGDfK)₂) | 607.478 | 4563.417 |
| DSPE-PEG3400- cyclo-GRENYHGCTTHWGFTLC-NH₂) | 607.818 | 5271.160 |
| cyclo-Ac-GRENYHGCTTHWGFTLCK-NH₂-(PEG3400-DSPE) | 607.480 | 5438.789 |
| DSPE-PEG3400- cyclo- K(DOTA)RENYHGCTTHWGFTLCNH₂ | 607.652 | 5855.646 |
| DSPE-PEG3400-YQGDAHFDDDEL | 607.473 | 4669.543 |
| DSPE-PEG3400-bicyclo-YADGAC_{1- 8}PC₃₋₉FLLGCC | 607.482 | 4698.642 |

### References

1. Lasic, D., Ceh, B., Stuart, M., Guo, L., Frederik, P., and Barenholz, Y. Biochim. Biophys. Acta 1239: 145-156, 1995
2. Tardi, P., Boman, N., and Cullis, P. J. Drug Target. 4: 129-140, 1996.
3. Sears, B. D. (1984). US Patent 4,426,330.
4. a) Storm, G., and Crommelin, D. Pharm. Sci. & Tech. Today 1: 19-31, 1998. b) Dagar, S., Sekosan, M., Lee, B., Rubinstein, I., and Önyüksel, H. J. of Contr. Rel. 74: 129-134, 2001. c) Penate Medina, O., Söderlund, T., Laakkonen, L., Tuominen, E., Koivunen, E., and Kinnunen, P. Cancer Res. 61: 2978-2985, 2001.
5. Torchilin, V.P. Nat. Rew. Drug Disc.145-160, 2005.
6. a) Torchilin, V.P., Rammohan, R., Weissig, V., Levchenko, T.S. Proc. Of Nat. Acad. Of Scien. 98: 8786-8791, 2001. b) Maeda, N., Takeuchi, Y., Takada, M., Namba, Y., Oku, N. Bioorg. Med. Chem. Lett. 14: 1015-1017, 2004. c) Decicco, C.P., Nelson, D.J., Barrett, J.A., Carpenter A.J., Duran, J.J., Rajopadhye, M. Patent WO 0160820
7. a) Tseng, Y-L., Liu, J-J., Hong, R-L. Mol. Pharm. 62:864-872, 2002. b) Schiffelers, R.M., Koning, G.A., ten Hagen, T.L.M., Fens, M.H.A.M., Schraa, A.J., Janssen, A.P.C.A., Kok, R.J., Molema, G., Storm, G. Jour. Of Controll. Rel. 91: 115-122, 2003.
8. a)Jensen, T.V., Hu, B-H., Delatore, S.M., Garcia, A.S., Messersmith, P. B., Miller, W.M. J. Am. Chem. Soc.126: 15223-15230, 2004. b) Wu, S-K. (2003) EP 1319667 A2
9. Carpenter, A. P. (2001). WO 01/60416 A2.
10. Zhu, Y. (2005) WO 2005/037862 A1
11. Koivunen, E., Ranti, T-M., Annila, A., Taube, S., Uppala, A., Jokinen, M., van Villigen, G., Ihanus, E., Gahmberg, C.G. J. Cell Biol. 153:905-915, 2001
12. Stefanidakis, M., Björklund, M., Ihanus, E., Gahmberg, C.G. J. Biol. Chem. 278:34674-34684, 2003
13. Zhu, L., Parr, G.R., Fitzgerald, M.C., Nelson, C.M., Smith, L.M. J. Am. Chem. Soc. 117:6048-6056, 1995

## Claims

1. A method of preparing a phospholipids-PEG-biomolecule conjugate comprising the steps of coupling pegylated phospholipids and a biomolecule by covalent attachment and purifying the obtained conjugate, wherein
- pegylated phospholipids are used in excess compared to the amount of the biomolecule,
- the coupling step is accelerated by the addition of inorganic additives, and
- the obtained phospholipids-PEG-biomolecule conjugate is purified by precipitation procedure.

2. The method according to claim 1, wherein the inorganic additives are a mixture of an inorganic base and an inorganic drying agent.

3. The method according to claim 2 wherein the inorganic base is selected from the group consisting of carbonates and bicarbonates of alkali metals, alkaline earth metals and lanthanide metals.

4. The method according to claim 3, wherein the alkali and alkaline earth metal carbonates are lithium carbonate, sodium carbonate and potassium carbonate.

5. The method according to claim 2 wherein the inorganic drying agent is selected from the group consisting of sulfates of alkali metals and alkaline earth metals.

6. The method according to claim 5 wherein the sulfates are sodium sulfate and magnesium sulfate.

7. The method according to claim 1 wherein the inorganic additives are used in excess compared to the amount of the biomolecule.

8. The method according to claim 1 wherein the precipitation comprises the steps of
(a) adding a suitable solvent or a mixture of solvents to the reaction mixture,
(b) dissolving the reaction material in a suitable alcohol,
(c) treating the dissolved reaction mixture with a solvent or a mixture of solvents to obtain a phospholipids-PEG-biomolecule precipitate,
(d) isolating the precipitate and, optionally, washing with solvent(s) and reisolating,
(d) optionally repeating the precipitation procedure from the alcohol solution, and
(e) optionally, dissolving the purified product in a suitably buffered water solution, freezing and lyophilizing.

9. The method according to claim 8, wherein the precipitation step from the alcohol solution is performed by a solvent or a mixture of solvents which forms one phase with the alcohol and is suitably hydrophobic to precipitate the phospholipids-PEG-biomolecule conjugate.

10. The method according to claim 9, wherein the solvent or the mixture of solvents comprises alkylether(s).

11. The method according to claim 10 wherein the solvent or the mixture of solvents comprises diethyl ether.

12. The method according to claim 8 wherein the alcohol is selected from the group consisting of methanol, ethanol, n-propanol, i-propanol, n-butanol, 2-butanol or t-butanol.

13. The method according to claim 1 wherein the biomolecule is a peptide selected from the group consisting of (E-cyclo-(RGDfK)₂), GRENYHGCTTHWGFTLC-NH₂, K(DOTA)RENYHGCTTHWGFTLC-NH₂, Ac-GRENYHGCTTHWGFTLCK-NH₂, YQGDAHGDDDEL and YADGAC₁₋₈PC₃₋₉FLLGCC.

14. The method according to any one of the preceding claims wherein the purity of the obtained phospholipid-PEG-biomolecule conjugate is analyzed chromatographically after derivatization of the phospholipid-PEG-biomolecule conjugate.

15. The method according to claim 14 wherein the derivatization of the phospholipids-PEG-biomolecule conjugate comprises basic hydrolysis of diacylesters to obtain hydrolyzed residual pegylated biomolecule.
